# EUROPÄISCHE PATENTSCHRIFT

(11) **EP 0 547 420 B1**
(45) Veröffentlichungstag und Bekanntmachung des Hinweises auf die Patenterteilung: **28.12.1994**
(21) Anmeldenummer: 92120450.9
(22) Anmeldetag: 01.12.1992
(51) Int. Cl.: C07C 13/20, C07C 1/213

(54) **Verfahren zur kontinuierlichen Gewinnung von Cyclohexen aus Cyclohexylestern**
Process for continuously obtaining cyclohexene from cyclohexyl esters
Procédé pour l'obtention en continu de cyclohexène à partir d'esters de cyclohexyle

(30) Priorität: 18.12.1991 DE 4141762
(43) Veröffentlichungstag der Anmeldung: 23.06.1993
(73) Patentinhaber: BASF Aktiengesellschaft, 67063 Ludwigshafen (DE)
(72) Erfinder: Pinkos, Rolf, Dr., W-6702 Bad Duerkheim (DE); Fischer, Rolf, Dr., W-6900 Heidelberg (DE); Dostalek, Roman, Dr., W-6725 Roemerberg (DE)

(56) Entgegenhaltungen:
- FR-A- 2 029 502
- US-A- 3 530 198
- CHEMISTRY LETTERS 1990, TOKYO JP Seiten 1201 - 1202 T. OKUHARA ET AL 'A pronounced Catalytic Activity of an acidic Cesium Salt of 12-Tungstophosphoric Acid for ester Decomposition in Solid-liquid System'
- JOURNAL OF CATALYSIS Bd. 78, Nr. 1, November 1982, NEW YORK Seiten 197 - 208 J. M: ADAMS ET AL 'Catalyzed Reactions of Organic Molecules at Clay Surfaces: Ester Breakdown, Dimerizations, and Lactonizations'

## Beschreibung

Die Patentanmeldung betrifft ein Verfahren zur Gewinnung von Cyclohexen durch Spaltung von Cyclohexylestern von Carbonsäuren mit 1 oder 2 Kohlenstoffatomen.

Aus Houben-Weyl, Methoden der organischen Chemie, Band 5/1b, 1972, Seite 105 ist bekannt, daß man Ester durch Thermolyse in der Gasphase bei Temperaturen über 350°C in die entsprechende Carbonsäure und Olefin spalten kann. Aus Journal of Cat., Band 78, Seite 197 (1982) ist zu entnehmen, daß Cyclohexylacetat, gelöst in Xylol bei einer Temperatur von 140°C in Gegenwart von Aluminiummontmorillonit in Cyclohexen und Essigsäure gespalten wird. Auch in Chem. Letters, 1990, Seite 1201 wird beschrieben, daß man Cyclohexylacetat gelöst in Xylol in Gegenwart von sauren Ionenaustauschern, Zeolithen in der H-Form oder Heteropolysäuren bei 100°C in Cyclohexen und Essigsäure spaltet. Dort wird darauf hingewiesen, daß Wasser für die Reaktion schädlich ist und deshalb die Katalysatoren vor dem Gebrauch getrocknet werden müssen, da in Gegenwart von Wasser die Säurestärke deutlich abnimmt. Aus keiner der Literaturstellen ist zu entnehmen, wie zu verfahren ist, um Cyclohexylester kontinuierlich in Cyclohexen und die entsprechende Säure zu spalten und wie die Spaltprodukte auf einfache Weise zu trennen sind.

Es war deshalb die technische Aufgabe gestellt, ein Verfahren zur kontinuierlichen Gewinnung von Cyclohexen aus Cyclohexylestern zur Verfügung zu stellen, das mit hohen Ausbeuten und Selektivitäten verläuft, bei dem Wasser nicht zu einer Verminderung der Aktivität der Katalysatoren führt und bei dem eine einfache und wirkungsvolle Trennung von Cyclohexen und Carbonsäure bewerkstelligt wird.

Diese Aufgabe wird gelöst in einem Verfahren zur kontinuierlichen Gewinnung von Cyclohexen aus Cyclohexylestern von Carbonsäuren mit 1 oder 2 Kohlenstoffatomen, welches folgende Schritte umfaßt:
a) Erhitzen von Cyclohexylestern von Carbonsäuren mit 1 oder 2 Kohlenstoffatomen in Gegenwart von sauren Katalysatoren unter Bildung von Cyclohexen und einer Carbonsäure mit 1 oder 2 Kohlenstoffatomen.
b) Abdestillieren von Cyclohexen und Carbonsäuren mit 1 oder 2 Kohlenstoffatomen in dem Maße, wie sie entstehen aus dem Reaktionsgemisch der Stufe a).
c) Zuführen von Cyclohexylester von Carbonsäuren mit 1 oder 2 Kohlenstoffatomen in die Stufe a), in dem Maße wie Cyclohexen und Carbonsäure abdestilliert werden und
d) Trennen von Cyclohexen und Carbonsäure mit 1 oder 2 Kohlenstoffatomen aus der Stufe b).

Das neue Verfahren hat den Vorteil, daß es kontinuierlich verläuft und leicht in einen technischen Maßstab übertragbar ist. Weiterhin hat das neue Verfahren den Vorteil, daß mit Cyclohexanol verunreinigter Cyclohexylester ohne vorherige Abtrennung des Cyclohexanols in Cyclohexen gespalten werden kann, ohne daß eine Verminderung der katalytischen Aktivität feststellbar ist. Ferner hat das neue Verfahren den Vorteil, daß die Trennung von Cyclohexen und Carbonsäure auf einfache Weise erfolgt.

Als Ausgangsstoff verwendet man Cyclohexylester von Carbonsäuren mit 1 oder 2 Kohlenstoffatomen. Das sind Cyclohexylformiat und Cyclohexylacetat sowie deren Gemische. Ferner können die als Ausgangsstoff verwendeten Cyclohexylester bis zu 30 Gew.-% Cyclohexanol enthalten. Solche Gemische aus Cyclohexylacetat oder -formiat mit Cyclohexanol fallen z.B. bei der Abtrennung von Cyclohexen aus Gemischen, die bei der partiellen Hydrierung von Benzol entstehen, an.

In einer ersten Stufe a) werden Cyclohexylester von Carbonsäuren mit 1 oder 2 Kohlenstoffatomen in Gegenwart von sauren Katalysatoren unter Bildung von Cyclohexen und einer Carbonsäure mit 1 oder 2 Kohlenstoffatomen erhitzt.

Als saure Katalysatoren verwendet man vorteilhaft Zeolithe in der H-Form, saure Ionenaustauscher, Heteropolysäuren, ferner saure und supersaure Metalloxide.

Bevorzugt werden Zeolithe der Mordenitgruppe oder engporige Zeolithe vom Erionit-Typ, Chabasit-Typ oder Zeolithe vom Faujasit-Typ, z.B. X-, Y- oder L-Zeolithe verwendet. In diese Gruppe gehören auch die sogenannten ultrastabilen Zeolithe des Faujasit-Typs, d.h. dealuminierte Zeolithe. Besonders vorteilhaft unter den Zeolithen sind solche mit Pentasilstruktur, wie ZSM 5, ZSM 11 und ZBM 10. Diese haben als Grundbaustein einen aus SiO₂-Tetraedern aufgebauten Fünfring gemeinsam. Sie sind durch ein hohes SiO₂/Al₂O₃-Verhältnis gekennzeichnet, sowie durch Porengrößen, die zwischen denen der Zeolithe vom Typ A und Typ X oder Y liegen.

Vorteilhaft verwendet man ferner als saure Katalysatoren anorganische Heteropolysäuren, die mindestens zwei verschiedene Zentralatome besitzen. Als Beispiele seien genannt: Dodecawolframatophosphorsäure, Dodecamolybdatophosphorsäure. Bevorzugt Heteropolysäuren sind Heteropolysäuren von Molybdän oder Wolfram mit Phosphorsäure, Tellursäure, Selensäure, Arsensäure, Kieselsäure, insbesondere mit Phosphorsäure. Die Protonen der Heteropolysäuren können teilweise durch Metallionen ersetzt sein, bevorzugt sind dabei Alkali- und Erdalkalimetallionen.

Ferner eignen sich saure Ionenaustauscher, z.B. vernetzte Polystyrole mit Sulfonsäuregruppen als saure Katalysatoren.

Ferner sind als saure Katalysatoren geeignet saure Metalloxide wie Siliciumdioxid, Aluminiumoxid, Zirkondioxid, Titandioxid, Zinndioxid, die zur Verstärkung auch mit Mineralsäure wie Schwefelsäure behandelt werden können.

Ferner sind als Katalysatoren geeignet Mineralsäuren wie Schwefelsäure oder Phosphorsäure, ferner organische Säuren wie Sulfonsäuren, z.B. Benzol- oder Toluolsulfonsäure.

Vorteilhaft wendet man je kg Katalysator und Stunde 5 bis 700 kg, insbesondere 50 bis 400 kg Cyclohexylester von Carbonsäuren mit 1 oder 2 Kohlenstoffatomen, gegebenenfalls zusammen mit Cyclohexanol an.

Es hat sich als vorteilhaft erwiesen, Verweilzeiten von 1 Minute bis 180 Minuten, vorzugsweise von 3 bis 90 Minuten, insbesondere von 5 Minuten bis 60 Minuten einzuhalten. Vorzugsweise hält man eine Temperatur von 50 bis 200°C, vorteilhaft von 70 bis 180°C, insbesondere von 90 bis 150°C ein. Es hat sich bewährt, wenn die Temperatur so gewählt wird, daß die Spaltprodukte leicht abdestillieren können. Es ist möglich, die Umsetzung auch bei Normaldruck oder schwach erhöhtem Druck oder schwach vermindertem Druck, z.B. von 0,1 bis 10 bar, insbesondere 0,3 bis 5 bar, besonders bevorzugt von 0,8 bis 2 bar durchzuführen.

In Schritt b) werden Cyclohexen und Carbonsäure, d.h. Essigsäure oder Ameisensäure, in dem Maße, wie sie entstehen, aus dem Reaktionsgemisch abdestilliert. Das Reaktionsgemisch besteht im wesentlichen aus den vorgenannten Estern des Cyclohexanols, ferner etwas Cyclohexanol, dem eingesetzten Katalysator sowie den Spaltprodukten Cyclohexen und Essigsäure oder Ameisensäure, sowie gegebenenfalls Wasser.

Als Schritt c) werden Cyclohexylester von Carbonsäure mit 1 oder 2 Kohlenstoffatomen, gegebenenfalls zusammen mit Cyclohexanol in dem Maße, wie Cyclohexen und Carbonsäure abdestilliert werden, der Stufe a) zugeführt.

Im Schritt d) werden Cyclohexen und Carbonsäuren mit 1 oder 2 Kohlenstoffatomen aus dem Schritt b) z.B. durch Destillation oder Phasentrennung getrennt.

Sofern man von Cyclohexylacetat ausgeht, ist es zweckmäßig, das abdestillierende Gemisch aus Cyclohexen und Essigsäure, gegebenenfalls zusammen mit Wasser in einer Kolonne zu trennen. Am Kopf der Kolonne wird Cyclohexen in reiner Form gewonnen, während Essigsäure, gegebenenfalls zusammen mit Wasser im Verlauf der Kolonne, als Seitenstrom abgezogen wird.

Nach einer bevorzugten Arbeitsweise werden die in Schritt b) anfallenden Brüden aus Cyclohexen, Ameisensäure oder Essigsäure und gegebenenfalls Wasser, kondensiert und gegebenenfalls durch Zugabe von Wasser in eine im wesentlichen aus Cyclohexen bestehende Phase und in eine im wesentlichen aus Wasser und Ameisensäure oder Essigsäure bestehende Phase getrennt. Aus der Cyclohexenphase wird durch Destillation reines Cyclohexen erhalten, während die gegebenenfalls wäßrige Carbonsäure z.B. wiederum bei der Abtrennung von Cyclohexen aus dem Benzolhydriergemisch verwendet werden kann.

Cyclohexen, wie es nach dem Verfahren nach der Erfindung erhältlich ist, eignet sich z.B. zur Herstellung von Cyclohexenoxid.

Das Verfahren nach der Erfindung sei an folgenden Beispielen erläutert.

### Beispiel 1

In einem 100 ml Reaktionsgefäß wurden 1 g H_{0,5}Cs_{2,5} PW₁₂O₄₀·xH₂O und 50 ml Cyclohexylformiat vorgelegt. Unter Rühren und Inertgas wurde auf 115°C aufgeheizt und über eine Kolonne Reaktionsprodukt abdestilliert. Die Menge an Destillat wurde kontinuierlich durch Zufahren von frischem Cyclohexylformiat in das Reaktionsgefäß ersetzt. Die Temperatur des Destillates im Kolonnenkopf betrug 73°C (1013 mbar). Das Destillat zerfiel in einem Auffanggefäß in zwei Phasen. Auf diese Weise wurden innerhalb 8 h insgesamt 970 g Cyclohexylformiat umgesetzt. Die Verweilzeit betrug ca. 0,5 h. Bei Abbruch der Reaktion war noch keine Desaktivierung festzustellen. Die Phasen des Reaktionsaustrages wurden portionsweise getrennt und auf ihre Zusammensetzung hin analysiert.

Es ergaben sich für die obere Phase folgende Zusammensetzungen:
Der Cyclohexengewichtsanteil schwankte zwischen 95 und 98 %, der Ameisensäuregewichtsanteil zwischen 2 und 4 %. Der Rest (maximal 1 %) war Cyclohexylformiat.

Für die untere Phase ergaben sich folgende Zusammensetzungen:
Der Cyclohexengewichtsanteil schwankte zwischen 1 und 2 %, der Ameisensäuregewichtsanteil zwischen 98 und 99 %. In geringen Anteilen (< 0,5 %) fand sich auch Cyclohexylformiat.

Im Reaktionssumpf konnten neben Cyclohexylformiat, Cyclohexen und Ameisensäure geringe Mengen Cyclohexylcyclohexen und Spuren an Cyclohexylcyclohexanol gefunden werden. Diese Nebenkomponenten waren aber auch schon im eingesetzten Cyclohexylformiat vorhanden.

Die Selektivität bezüglich Cyclohexen und Ameisensäure betrug > 99 %, bezogen auf eingesetztes Cyclohexylformiat.

### Beispiel 2

Analog Beispiel 1 wurden 500 g Cyclohexylacetat mit 1 g Wolframatophosphorsäure umgesetzt. Bei Verweilzeiten von 0,4 bis 0,6 h und einer Sumpftemperatur von 121 bis 125°C und Kopftemperaturen von 87 bis 98°C wurde ein einphasiger Reaktionsaustrag erhalten. Dieser wies einen Cyclohexengewichtsanteil von 55 bis 65 % und einen Essigsäuregewichtsanteil von 35 bis 45 % auf. Mitunter wurden geringe Mengen Cyclohexylacetat gefunden (< 0,5 %). Die Selektivitäten bezüglich Cyclohexen und Essigsäure, bezogen auf eingesetztes Cyclohexylacetat, lagen > 99 %.

Von den vereinigten Reaktionsausträgen wurden 100 g mit 1,6 g Wasser versetzt. Es trat eine sofortige Phasentrennung auf, wobei in der unteren Phase (ca. 20 g) 80 % Gewichtsanteil Essigsäure zu finden war.

### Beispiel 3

Analog Beispiel 1 wurden 277 g eines Gemisches aus 80 Mol-% Cyclohexylacetat und 20 Mol-% Cyclohexanol mit 1 g Wolframatophosphorsäure umgesetzt. Bei Verweilzeiten von 0,6 h wurden bei Sumpftemperaturen von 125°C und Kopftemperaturen von ca. 96°C zweiphasige Austräge folgender Zusammensetzung erhalten:

Die obere Phase wies Cyclohexengewichtsanteile von durchschnittlich 82 %, Essigsäuregewichtsanteile von durchschnittlich 17 % auf. Der Rest bestand jeweils aus Cyclohexylacetat und Wasser.

Die untere Phase wies Cyclchexengewichtsanteile von 4 bis 10 %, Essigsäuregewichtsanteile von 80 bis 85 % auf. Der Rest bestand jeweils aus Wasser sowie geringen Mengen Cyclohexylacetat.

Die Selektivität bezüglich Cyclohexen und Essigsäure betrug > 99 %, bezogen auf eingesetztes Cyclohexylacetat/Cyclohexanol.

## Patentansprüche

1. Verfahren zur kontinuierlichen Gewinnung von Cyclohexen aus Cyclohexylestern von Carbonsäuren mit 1 oder 2 Kohlenstoffatomen, welches folgende Schritte umfaßt:
a) Erhitzen von Cyclohexylestern von Carbonsäuren mit 1 oder 2 Kohlenstoffatomen in Gegenwart von sauren Katalysatoren unter Bildung von Cyclohexen und einer Carbonsäure mit 1 oder 2 Kohlenstoffatomen.
b) Abdestillieren von Cyclohexen und Carbonsäure mit 1 oder 2 Kohlenstoffatomen in dem Maße, wie sie entstehen aus dem Reaktionsgemisch der Stufe a).
c) Zuführen von Cyclohexylester von Carbonsäuren mit 1 oder 2 Kohlenstoffatomen in die Stufe a), in dem Maße wie Cyclohexen und Carbonsäure abdestilliert werden und
d) Trennen von Cyclohexen und Carbonsäure mit 1 oder 2 Kohlenstoffatomen aus der Stufe b).

2. Verfahren nach Anspruch 1, dadurch gekennzeichnet, daß man als saure Katalysatoren Zeolithe, saure Ionenaustauscher, Heteropolysäuren, saure oder supersaure Metalloxide verwendet.

3. Verfahren nach den Ansprüchen 1 und 2, dadurch gekennzeichnet, daß man 5 bis 700 kg Cyclohexylester von Carbonsäuren mit 1 oder 2 Kohlenstoffatomen je kg Katalysator und Stunde einsetzt.

4. Verfahren nach den Ansprüchen 1 bis 3, dadurch gekennzeichnet, daß man in Stufe eine Temperatur von 90 bis 150°C einhält.

5. Verfahren nach den Ansprüchen 1 bis 4, dadurch gekennzeichnet, daß man in der Stufe a) eine Verweilzeit von 3 bis 90 Minuten einhält.

6. Verfahren nach den Ansprüchen 1 bis 5, dadurch gekennzeichnet, daß man Cyclohexen und Carbonsäuren aus Schritt b) kondensiert, das Kondensat mit Wasser versetzt und eine im wesentlichen aus Cyclohexen bestehende Phase von der im wesentlichen aus Wasser und Carbonsäuren mit 1 oder 2 Kohlenstoffatomen bestehende Phase abtrennt.

7. Verfahren nach den Ansprüchen 1 bis 5, dadurch gekennzeichnet, daß man bis zu 30 Gew.-% Cyclohexanol, bezogen auf Cyclohexylester von Carbonsäure mit 1 oder 2 Kohlenstoffatomen mitverwendet.

8. Verfahren nach den Ansprüchen 1 bis 5 und 7, dadurch gekennzeichnet, daß man bei der Spaltung von Cyclohexylacetat, das Cyclohexanol enthalten kann, in Stufe b das Gemisch aus Cyclohexen und Essigsäure und gegebenenfalls Wasser in einer Kolonne trennt, wobei man Cyclohexen am Kopf der Kolonne und Essigsäure sowie gegebenenfalls Wasser im Verlauf der Kolonne seitlich entnimmt.

## Claims

1. A process for the continuous production of cyclohexene from cyclohexyl esters of carboxylic acids of 1 or 2 carbon atoms, comprising the following steps:
a) heating cyclohexyl esters of carboxylic acids of 1 or 2 carbon atoms in the presence of acidic catalysts to form cyclohexene and a carboxylic acid of 1 or 2 carbon atoms,
b) distilling cyclohexene and the carboxylic acid of 1 or 2 carbon atoms out of the reaction mixture of stage a) at the rate at which they are formed,
c) feeding cyclohexyl esters of carboxylic acids of 1 or 2 carbon atoms into stage a) at the rate at which cyclohexene and carboxylic acid are distilled off, and
d) separating cyclohexene and the carboxylic acid of 1 or 2 carbon atoms from stage b).

2. A process as claimed in claim 1, wherein the acidic catalysts used are zeolites, acidic ion exchangers, heteropoly acids, or acidic or superacidic metal oxides.

3. A process as claimed in claim 1 or 2, wherein from 5 to 700 kg of cyclohexyl esters of carboxylic acids of 1 or 2 carbon atoms are used per kg of catalyst per hour.

4. A process as claimed in any of claims 1 to 3, wherein stage a) is carried out at from 90 to 150°C.

5. A process as claimed in any of claims 1 to 4, wherein stage a) is carried out with a residence time of from 3 to 90 minutes.

6. A process as claimed in any of claims 1 to 5, wherein cyclohexene and carboxylic acids from step b) are condensed, water is added to the condensate, and a phase consisting essentially of cyclohexene is separated from the phase consisting essentially of water and carboxylic acids of 1 or 2 carbon atoms.

7. A process as claimed in any of claims 1 to 5, wherein up to 30% by weight of cyclohexanol, based on cyclohexyl esters of carboxylic acids of 1 or 2 carbon atoms, is included.

8. A process as claimed in any of claims 1 to 5 and 7, wherein in the cleavage of cyclohexyl acetate with or without cyclohexanol the mixture of cyclohexene and acetic acid with or without water is separated in stage b) in a column, the cyclohexene being withdrawn at the top of the column and the acetic acid and the water, if any is present, being withdrawn at the side of the column.

## Revendications

1. Procédé pour l'obtention en continu de cyclohexène à partir d'esters cyclohexyliques d'acides carboxyliques à 1 ou 2 atomes de carbone, comprenant les étapes suivantes:
a) Chauffage d'esters cyclohexyliques d'acides carboxyliques à 1 ou 2 atomes de carbone, en présence de catalyseurs acides, avec formation de cyclohexène et d'un acide carboxylique à 1 ou 2 atomes de carbone;
b) Elimination par distillation de cyclohexène et d'acide carboxylique à 1 ou 2 atomes de carbone, dans la mesure où ils se forment à partir du mélange réactionnel de l'étape a);
c) Apport d'ester cyclohexylique d'acide carboxylique à 1 ou 2 atomes de carbone dans l'étape a), dans la mesure où le cyclohexène et l'acide carboxylique sont chassés par distillation, et
d) Séparation du cyclohexène et de l'acide carboxylique à 1 ou 2 atomes de carbone provenant de l'étape b).

2. Procédé selon la revendication 1, caractérisé en ce qu'on utilise, comme catalyseurs acides, des zéolithes, des échangeurs d'ions acides, des hétéropolyacides ou des oxydes métalliques acides ou superacides.

3. Procédé selon la revendication 1 ou 2, caractérisé en ce qu'on met en réaction, par kg de catalyseur et par heure, de 5 à 700 kg d'esters cyclohexyliques d'acides carboxyliques à 1 ou 2 atomes de carbone.

4. Procédé selon l'une quelconque des revendications 1 à 3, caractérisé en ce qu'on maintient, dans l'étape a), une température de 90 à 150°C.

5. Procédé selon l'une quelconque des revendications 1 à 4, caractérisé en ce qu'on maintient, dans l'étape a), une durée de séjour de 3 à 90 mn.

6. Procédé selon l'une quelconque des revendications 1 à 5, caractérisé en ce qu'on condense le cyclohexène et les acides carboxyliques provenant de l'étape b), on mélange le condensat avec de l'eau et on sépare une phase composée essentiellement de cyclohexène d'avec la phase composée essentiellement d'eau et d'acides carboxyliques à 1 ou 2 atomes de carbone.

7. Procédé selon l'une quelconque des revendications 1 à 5, caractérisé en ce qu'on utilise simultanément jusqu'à 30% en poids de cyclohexanol, par rapport aux esters cyclohexyliques d'acide carboxylique à 1 ou 2 atomes de carbone.

8. Procédé selon l'une quelconque des revendications 1 à 5 et 7, caractérisé en ce que, dans le cas de la dissociation d'acétate de cyclohexyle, qui peut contenir du cyclohexanol, on sépare dans l'étape b) le mélange de cyclohexène, d'acide acétique et éventuellement d'eau dans une colonne, le cyclohexène étant prélevé en tête de la colonne, tandis que l'acide acétique et éventuellement l'eau sont extraits latéralement le long de la colonne.
